(19) Europäisches Patentamt
European Patent Office
Office européen des brevets

(11) **EP 4 764 588 A1**

(12) **EUROPEAN PATENT APPLICATION**

(43) Date of publication:
24.06.2026 Bulletin 2026/26

(21) Application number: 24307139.6

(22) Date of filing: **17.12.2024**

(51) International Patent Classification (IPC):
**G01S 5/16** (2006.01)   **A61B 5/06** (2006.01)
**A61B 34/20** (2016.01)   **G01V 3/10** (2006.01)

(52) Cooperative Patent Classification (CPC):
**G01S 5/16; A61B 5/062;** A61B 2034/2051

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **MinMaxMedical
38400 Saint-Martin-d'Hères (FR)**

(72) Inventors:
• HUGUEL, Loïc
  38400 Saint-Martin-d'Hères (FR)
• ROUSSEAU, Sandra
  38400 Saint-Martin-d'Hères (FR)
• REYMOND, Solveig
  38400 Saint-Martin-d'Hères (FR)

(74) Representative: **Regimbeau
20, rue de Chazelles
75847 Paris Cedex 17 (FR)**

(54) **METHOD FOR ELECTROMAGNETICALLY LOCALIZING A RECEIVER**

(57)    The present disclosure concerns a method for electromagnetically localizing a receiver (20) with respect to a transmitter (10), comprising:
- emitting (E1) a respective magnetic field by each magnetic field generator (11) of the transmitter (10);
- receiving and measuring (E2) each emitted magnetic field using the receiver (20); and
- calculating (E45, E5) a position of the receiver (20) based on each measured magnetic field.

**FIG. 1**

**Description**

TECHNICAL FIELD

The present disclosure relates to the field of electromagnetic localization of a receiver with respect to a transmitter.

TECHNICAL BACKGROUND

Computer-assisted surgery, surgical navigation, and surgical robotics all rely on the measurement of the relative position and/or orientation between several trackers attached to anatomical structures or bones, to surgical instruments, to robot ends, or to various sensors.

Optical localization systems are well known and widely used, though limited by the bulkiness of their large trackers and line of sight major constraints. Electromagnetic systems are particularly adapted to be used during minimally invasive surgeries, as they allow an electromagnetic transducer to be inserted into the patient through an incision, and as they are not limited by any line-of-sight issue.

An electromagnetic localization device generally consists of a transmitter comprising one or several transmitting coils rigidly linked to each other, and of a receiver comprising one or several receiving coils rigidly linked to each other. As well known in the art, the joint analysis and modeling of the magnetic fields emitted by the transmitting coils and measured by the receiving coils makes it possible to determine the pose, that is to say the position and/or orientation, of the receiver with respect to the transmitter. Consequently, the respective pose of objects linked to each of the receiver and the transmitter can be determined as long as the geometry of the links between said receiver or transmitter and the objects to be tracked are known.

Typically, a transmitter comprises three coils and a receiver comprises three smaller coils. Therefore, nine measurements are obtained, and the six independent degrees of freedom of the pose of a transform matrix between the receiver reference system and the transmitter reference system can be determined. In many systems, it is possible to use one transmitter and several receivers, in order to determine the relative poses of these receivers with respect to each other. For example, one receiver can be attached to a bone, and another one to the tip of an instrument, so that the relative pose of the bone with respect to the tip of the instrument can be determined.

Electromagnetic localization devices are well known in the literature, as for example in the document "Magnetic Position and Orientation Tracking System" by Frederick H. Raab et al. published in IEEE Transactions on Aerospace and Electronic Systems VOL. AES-15, No. 5 September 1979, or the document "La localisation spatiale d'outils chirurgicaux par systemes electromagnetiques alternatifs. Applications et domaines de validite des modelisations numeriques" by Joffrey Paille - Thesis, Universit6 Joseph-Fourier - Grenoble I, 2004. HAL Id: tel-00006125, version 1.

However, electromagnetic localization methods are confronted with magnetic dipole ambiguity. Indeed, the equations used to calculate distances based on the measured magnetic fields emitted by the transmitter may yield multiple solutions. Thus, the method may converge towards the real position of the receiver, that is to say the actual physical location of the receiver in space, but may also converge toward the mirror position of the receiver, that is to say an alternative, symmetrical solution to the real position with respect to a specific surface or point. This may lead to an inaccurate localization of the receiver with respect to the transmitter, thus an inaccurate localization of the medical tool or implant, which may as a result endanger the surgery.

Some systems solve this magnetic dipole ambiguity by implementing a transmitter comprising several coils and calculating, for each coil, all the potential positions of the receiver, in order to find the real position. However, these systems are costly in calculation time.

Other systems solve this magnetic dipole ambiguity by requesting the user to input a position information of the receiver before implementing the process. However, such systems are restrictive for the user and prone to human error.

Document US 8 180 430 B2 discloses a system for solving magnetic dipole ambiguity using multiple magnetic field generators and sensors and calculating corresponding potential positions. The system incorporates pre-determined positional information such as an arrow marking positioned on the location pad and indicating the direction of the generated magnetic field, or several location pads located at different distances of the object. Thus, the system can eliminate the mirror positions and determine the real position of the receiver. However, such a determination is costly in time, at it is necessary to position the arrow in the right direction on the location pad, or is costly in equipment, as at least two location pads are necessary to solve magnetic dipole ambiguity.

SUMMARY

An aim of the present disclosure is to propose a method for electromagnetically localizing a receiver which allows solving of the magnetic dipole ambiguity in a way which is accurate, reliable and less restrictive for the user.

According to a first aspect, the present disclosure relates to a method for electromagnetically localizing a receiver with respect to a transmitter, comprising:

- emitting a respective magnetic field by each magnetic field generator of the transmitter, said transmitter comprising at least four non-coplanar magnetic field generators separated from each other by known distances;

- receiving and measuring each emitted magnetic field using the receiver;
- calculating a respective norm for each respective measured magnetic field, each respective norm being representative of a distance between the receiver and the respective magnetic field generator;
- estimating a preliminary position of the receiver based on the calculated respective norms; and
- calculating a position of the receiver based on each measured magnetic field and on the estimated preliminary position.

The receiver may be a tri-axis orthogonal receiver.

The receiver may be a tri-axis orthonormal receiver.

The receiving coils of the receiver may be approximately concentric.

The transmitter may comprise at least a pair of two magnetic field generators positioned symmetrically with respect to a center of the transmitter.

The transmitter may comprise six magnetic field generators.

The transmitter may further comprise a housing in the form of a rectangular block, the six magnetic field generators of the transmitter may be arranged in three orthogonal opposing pairs, each magnetic field generator being positioned facing a respective side of the rectangular block of the housing.

The housing of the transmitter may be in the form of a cube.

Each known distance separating two magnetic field generators may be at least equal to 0,5 cm, for example may be comprised between 1 cm and 5 cm, for example may be equal to 3 cm.

Estimating the preliminary position of the receiver may comprise running a multilateration algorithm on the calculated norms of the measured magnetic fields.

Calculating the position of the receiver may comprise running a minimization algorithm starting at the estimated preliminary position of the receiver.

The method may be for electromagnetically localizing several receivers with respect to a transmitter, wherein each receiver receives and measures each emitted magnetic field, wherein a preliminary position is estimated for of each respective receiver and wherein a position of each respective receiver is calculated based on each measured magnetic field and on each respective estimated preliminary position.

The method may be for electromagnetically localizing a receiver with respect to several transmitters, wherein each of the several transmitters comprises at least four non-coplanar magnetic field generators separated from each other by known distances, and wherein the steps of calculating the respective norm and of calculating the preliminary position are performed for each of the several transmitters.

According to a second aspect, the present disclosure relates to a method for electromagnetically localizing a receiver with respect to a transmitter, comprising:

- emitting a respective magnetic field by each magnetic field generator of the transmitter, said transmitter comprising at least three non-coplanar magnetic field generators separated from each other by known distances;
- receiving and measuring each emitted magnetic field using the receiver;
- calculating a first position of the receiver based on each measured magnetic field and on a first initial position hypothesis;
- calculating a first residual corresponding to the first position;
- calculating a second position of the receiver based on each measured magnetic field and on a second initial position hypothesis, wherein the second initial position hypothesis corresponds to a mirror image of the first position of the receiver calculated in step;
- calculating a second residual corresponding to the second position; and
- selecting the position of the receiver associated with the smallest of the first residual and the second residual, as corresponding to the calculated position of the receiver.

The receiver may be a tri-axis orthogonal receiver.

The receiver may be a tri-axis orthonormal receiver.

The receiving coils of the receiver may be approximately concentric.

The transmitter may comprise at least a pair of two magnetic field generators positioned symmetrically with respect to a center of the transmitter.

The transmitter may comprise six magnetic field generators.

The transmitter may further comprise a housing in the form of a rectangular block, the six magnetic field generators of the transmitter may be arranged in three orthogonal opposing pairs, each magnetic field generator being positioned facing a respective side of the rectangular block of the housing.

The housing of the transmitter may be in the form of a cube.

Each known distance separating two magnetic field generators may be at least equal to 0,5 cm, for example may be comprised between 1 cm and 5 cm, for example may be equal to 3 cm.

Calculating the first position of the receiver may comprise running a minimization algorithm starting at the first initial position hypothesis and calculating the second position of the receiver may comprise running a minimization algorithm starting at the second initial position hypothesis.

The method may be for electromagnetically localizing several receivers with respect to a transmitter, wherein each receiver receives and measures each emitted magnetic field, wherein the first position of each respective receiver is calculated based on each measured magnetic field and on the first initial position hypothesis and wherein the second position of each respective receiver is calculated based on each measured magnetic field and on the second initial position hypothesis.

BRIEF DESCRIPTION OF THE DRAWINGS

Other features and advantages of the disclosure will appear in the following description with reference to the following figures.

Figure 1 illustrates, schematically, an exploded perspective view of a transmitter used to implement the method.
Figure 2 illustrates, schematically, a perspective view of a receiver used to implement the method.
Figure 3 illustrates, schematically, a system used to implement the method.
Figure 4 illustrates a block diagram of a method for electromagnetically localizing a receiver with respect to a transmitter according to a first aspect.
Figure 5 illustrates of a method for electromagnetically localizing a receiver with respect to a transmitter according to a second aspect.

DETAILED DESCRIPTION

Method for electromagnetically localizing a receiver

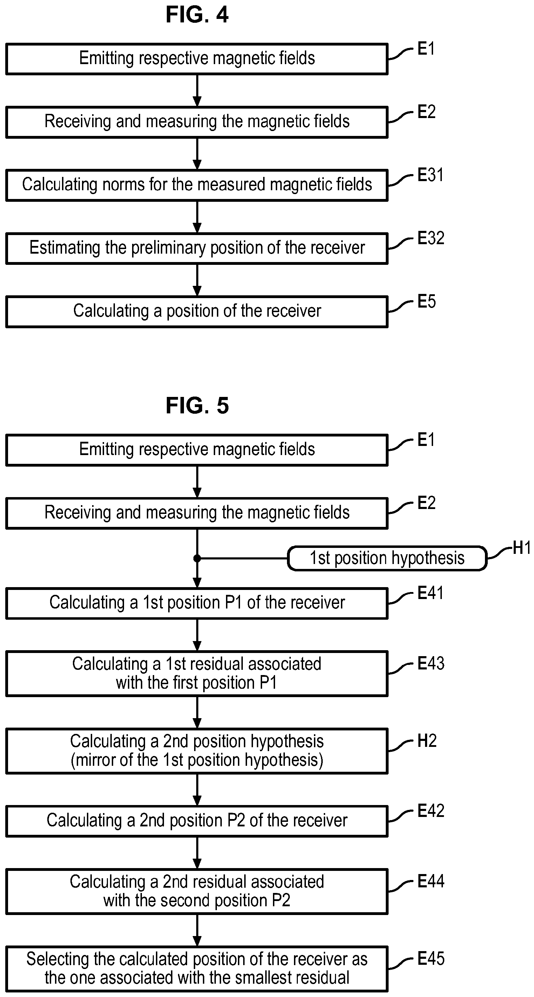

According to a first aspect, a method for electromagnetically localizing a receiver 20 with respect to a transmitter 10 is illustrated by way of a non-limiting example in figure 4. The method comprises:

- emitting E1 a respective magnetic field by each magnetic field generator 11 of the transmitter 10, said transmitter 10 comprising at least four non-coplanar magnetic field generators 11 separated from each other by known distances;
- receiving and measuring E2 each emitted magnetic field using the receiver 20;
- calculating E31 a respective norm for each respective measured magnetic field, each respective norm being representative of a distance between the receiver 20 and the respective magnetic field generator 11;
- estimating E32 a preliminary position of the receiver 20 based on the calculated respective norms; and
- calculating E5 a position of the receiver 20 based on each measured magnetic field and on the estimated preliminary position.

According to a second aspect, a method for electromagnetically localizing a receiver 20 with respect to a transmitter 10 is illustrated by way of a non-limiting example in figure 5. The method comprises:

- emitting E1 a respective magnetic field by each magnetic field generator 11 of the transmitter 10, said transmitter 10 comprising at least three non-coplanar magnetic field generators 11 separated from each other by known distances;
- receiving and measuring E2 each emitted magnetic field using the receiver 20;
- calculating E41 a first position P1 of the receiver 20 based on each measured magnetic field and on a first initial position hypothesis H1;
- calculating E43 a first residual corresponding to the first position P1;
- calculating E42 a second position P2 of the receiver 20 based on each measured magnetic field and on a second initial position hypothesis H2, wherein the second initial position hypothesis H2 corresponds to a mirror image of the first position P1 of the receiver 20 calculated in step E41;
- calculating E44 a second residual corresponding to the second position P2; and
- selecting E45 the position P1, P2 of the receiver 20 associated with the smallest of the first residual and the second residual, as corresponding to the calculated position of the receiver 20.

Each of the method according to the first aspect and to the second aspect allows solving of the magnetic dipole ambiguity in a way which is accurate, reliable and less restrictive for the user. More specifically, each of the method according to the first aspect and to the second aspect allows an accurate and reliable localizing of the receiver 20, without requiring pre-determined input by a user.

Furthermore, each of the method according to the first aspect and to the second aspect allows electromagnetically localizing the receiver 20 even when the receiver 20 is positioned far away, that is to say more than several centimeters away, from the transmitter 10. Thus, the position of the receiver 20 can be calculated accurately even when the receiver 20 is located far away from the transmitter 10.

The method according to the first aspect allows for obtaining a preliminary position of the receiver 20 which is located in a hemisphere where a real position of the receiver 20 is located, instead of a hemisphere where a mirror position of the receiver 20 is located. The real position of the receiver 20 corresponds to the actual physical position of the receiver 20 in space, which corresponds to the true location of the receiver 20 with respect to the transmitter 10. The mirror position of the receiver 20 corresponds to an alternative, symmetrical solution to the real position with respect to a specific surface or point. The mirror position is a secondary, incorrect solution which can arise during position calculation due to magnetic dipole ambiguity, as the equations used to calculate distances based on the measured magnetic fields emitted by the transmitter 10 may yield multiple solutions. With the method according to the first aspect, the preliminary position of the receiver 20 is representative of the hemisphere where the real position of the receiver 20 is located and is also representative of a distance between the receiver 20 and the transmitter 10. Therefore, the method according to the first aspect allows the position calculation E5 of the receiver 20 to start from an initial position which is located in the hemisphere where the receiver 20 is physically located. Therefore, the position calculation E5 does not risk converging towards a local minimum, but rather will converge towards the global minimum which corresponds to the real position of the receiver 20. Also, the position calculation E5 quickly converges towards the real position of the receiver 20, thus the method is less costly in calculation time and/or computing power.

Definitions

In the present document, the word "pose" is defined as the position and/or orientation of an object with respect to another object. It can for instance be represented as a transform matrix between a reference system attached to a first object and a reference system attached to the other object.

Localizing an object amounts to determining the pose of the concerned object, i.e. the position of the object for example carrying a magnetic transmitter 10, as well as its orientation or its inclination with respect to another object, from magnetic data transmitted by said magnetic field transmitter 10 and measured by receiver 20 carried by said other object. In the present case, as will be seen later, said object can be fixed or mobile.

Moreover, in the present description, the use of the word "magnetic" can be replaced by the word "electromagnetic" in the sense that the source - the magnetic transmitter 10 - can be electromagnetic technology, and the magnetic receiver 20 can also be electromagnetic technology.

Transmitter

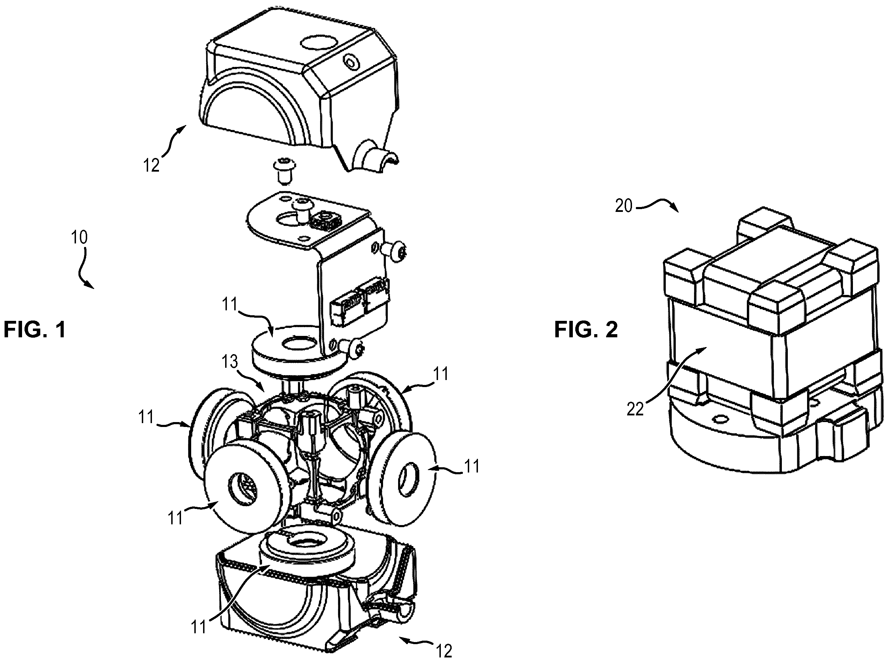

A system for implementing the electromagnetic localization method disclosed above may comprise a transmitter 10, as illustrated by way of a non-limiting example in figure 1. The transmitter 10 comprises at least four magnetic field generators 11 to implement the method according to the first aspect and at least three magnetic field generators 11 to implement the method according to the second aspect. Each magnetic field generator 11 may be a transmitting coil. The transmitting coil may have any suitable shape, and may for example be a circular coil, an elliptical coil, a square coil, a rectangular coil, etc.

The magnetic field generators 11 are non-coplanar. In other words, the magnetic field generators 11 may be oriented along distinct axes which may be parallel, intersecting, or perpendicular and/or the magnetic field generators 11 may have a same axis but have distinct centers. The center of a magnetic field generator 11 corresponds to a geometric center of the transmitting coil's 11 physical structure, for example may correspond to a barycenter of the transmitting coil's 11 windings. For example, in the case of a circular coil, the center of the magnetic field generator 11 corresponds to a point equidistant from all parts of the transmitting coil's 11 windings. More particularly, the transmitter 10 can comprise three orthogonal magnetic field generators 11, that is to say oriented along the three axes of an orthogonal reference frame 13.

The magnetic field generators 11 of the transmitter 10 may be rigidly connected to each other. For example, the transmitter 10 may comprise a frame 13 on which each magnetic field generators 11 is mounted. The frame 13 is a mechanical structure that holds the transmitting coils 11 together in a fixed orientation relative to each other. The frame 13 may include cube edges on which the magnetic field generators 11 are mounted, as illustrated by way of a non-limiting example in figure 1.

The transmitter 10 may comprise a housing 12 that contains the components of the transmitter 10, such as the magnetic field generators 11 and the frame 13. The housing 12 of the transmitter 10 may be in the form of a rectangular block or in the form of a cube.

The transmitter 10 may comprise at least a pair of two magnetic field generators 11 positioned symmetrically with respect to a center of the transmitter 10. The shape, size and/or arrangement of the at least two magnetic field generators 11 of the pair may be symmetrical with respect to the center of the transmitter 10. Such a pair of two symmetric magnetic field generators 11 improves the homogeneity of the accuracy of the localization of the receiver 20 with respect to the transmitter 10, whatever the localization of the receiver 20. More specifically, the at least two magnetic field generators 11 of the pair may be oriented along a same axis but have distinct centers separated from each other by a known distance. The transmitter 10 may comprise two pairs of two magnetic field generators 11 positioned symmetrically with respect to the center of the transmitter 10.

The transmitter 10 may comprise four, five, six, or more, magnetic field generators 11.

When the transmitter 10 comprises six magnetic field generators 11, the six magnetic field generators 11 may be arranged in three opposing pairs, each of the two magnetic field generators 11 of a given pair being positioned symmetrically with respect to a center of the transmitter 10. More specifically, the six magnetic field generators 11 may be arranged in three orthogonal opposing pairs. Each magnetic field generator 11 may be separated from the center of the transmitter 10 by a predetermined distance.

Thus, the transmitter 10 may comprise a housing 12 in the form of a rectangular block, the six magnetic field generators 11 of the transmitter 10 may be arranged in three orthogonal opposing pairs, each magnetic field generator 11 being positioned facing a respective side of the rectangular block of the housing 12. The frame 13 may be substantially cubic, each magnetic field generator 11 being mounted on a respective side of the frame 13.

The known distances separating the magnetic field generators 11 from each other may include several different known distances or may include only an identical distance if the magnetic field generators 11 are positioned equidistant from each other. The distances separating two magnetic field generators 11 are known from the initial configuration of the transmitter 10.

Each known distance separating two magnetic field generators 11 may be at least equal to 0,5 cm, for example may be comprised between 1 cm and 5 cm, for example may be equal to 3 cm. A distance separating each of the magnetic field generators 11 determines a maximum distance at which a receiver 20 may be located. The distances separating each of the magnetic field generators 11 disclosed above are sufficient to allow for accurate estimating of the preliminary position of the receiver 20 based on the calculated norms in the method according to the first aspect, even when the transmitter 10 and receiver 20 are positioned far away from each other, that is to say more than several centimeters away from each other. When the distance separating each of the magnetic field generators 11 is greater than 1 cm, the receiver 20 can be located even when it is located more than several tens of centimeters away from the transmitter 10.

The transmitter 10 may further comprise communicating means configured to communicate, for example wirelessly, with a processing unit.

### Receiver

The system for implementing the electromagnetic localization method disclosed above may comprise a receiver 20, as illustrated by way of a non-limiting example in figure 2. The receiver 20 may comprise a number of receiving coils 21, for example at least three receiving coils 21. Alternatively, the receiver 20 can comprise a number of magneto-resistive sensors, such as anisotropic magnetoresistance sensors, global magneto-resistive sensor, tunnel magneto-resistive sensors or SQUID (superconducting quantum interference device) sensors. As known in the art, such magneto-resistive sensors comprise a resistive element with a resistance proportional to a value of the magnetic field they receive.

The receiving coils 21 of the receiver 20 may be non-coplanar, that is to say do not lie in a same plane. The receiving coils 21 may be oriented along distinct axes, and/or be parallel to each other but spatially separated by a distance. More particularly, the receiver 20 may be a tri-axis orthogonal receiver, more particularly may be a tri-axis orthonormal receiver. The receiver 20 thus comprises three orthogonal receiving coils 21, that is to say oriented along the three axes of an orthogonal reference. Such a tri-axis orthogonal receiver 20 allows for measuring not only the position of the receiver 20 with respect to the transmitter 10, but also its orientation. Each receiving coil 21 measuring the component of the magnetic field along its respective axis ensure uniform measurement across all directions.

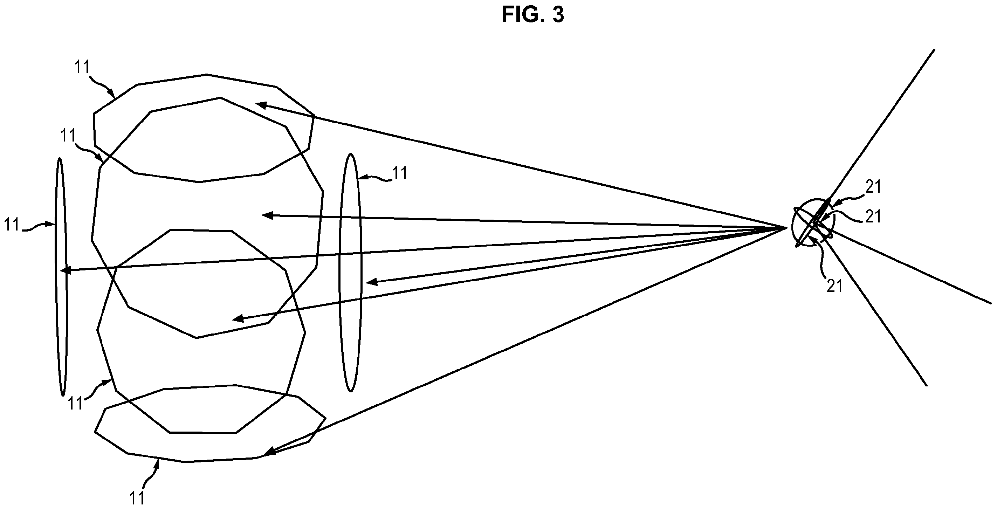

The receiving coils 21 of the receiver 20 may be approximately concentric, that is to say they approximately share a same center, as illustrated by way of a non-limiting example in figure 3. Therefore, the distance between a center of the receiving coil 21 to a given magnetic field generator 11 is approximately the same for all of the receiving coils 21. The more concentric the receiving coils 21, the better the accuracy of the localization.

The receiving coils 21 of the receiver 20 may be rigidly connected to each other. For example, the receiver 20 may comprise a frame on which each receiving coil 21 is mounted. The frame is a mechanical structure that holds the receiving coils 21 together in a fixed orientation relative to each other.

The receiver 20 may comprise a housing 22 that contains the components of the receiver 20, such as the receiving coils 21 and the frame. The housing 22 of the receiver 20 may be in the form of a rectangular block or in the form of a cube.

The receiver 20 may further comprise communicating means configured to communicate, for example wirelessly, with a processing unit. Alternatively, or in addition, the receiver 20 may further comprise a processing unit configured to calculate the position of the receiver 20 based on each measured magnetic field and on the estimated preliminary position.

The receiver 20 may further comprise a power supply providing electrical power to the communicating means of the receiver 20.

### Processing unit

The system for implementing the electromagnetic localization method disclosed above may comprise one or several processing unit(s) configured to implement one or several steps of the method according to the first aspect and/or the method according to the second aspect. For example, the processing unit(s) may be configured to estimate E32 the preliminary position of the receiver 20 and to calculate E5 the position of the receiver 20 based on each measured magnetic field and on the estimated preliminary position in the method according to the first aspect. The processing unit(s) may be configured to calculate E41 the first position P1 of the receiver 20 based on each measured magnetic field and on the first initial position hypothesis H1 and to calculate E42 the second position P2 of the receiver 20 based on each measured magnetic field and on the second initial position hypothesis H2 in the method according to the second aspect. The processing unit(s) may be configured to calculate E43 the first residual and to calculate E44 the second residual in the method according to the second aspect.

The processing unit may be integrated in the transmitter 10, in the receiver 20, and/or in an external controller. The processing unit may comprise communicating means configured to communicate, for example wirelessly, with the communicating means of the transmitter 10 and/or with the communicating means of the receiver 20.

The transmitter 10 and the receiver 20 are connected to the processing unit and can communicate with the processing unit, for example via their respective communicating means. The communication can be a wireless communication.

The processing unit may include for example a microprocessor connected on one hand to the magnetic field generators 11 of the transmitter 10 and on the other hand to the receiver 20. The processing unit is configured to process the signals transmitted and received, and thus makes it possible to determine from the latter the position and/or orientation of the receiver 20 relative to the transmitter 10.

The processing unit receives information concerning the characteristics of the signals passing through the receiving coils 21. From the intensity of the magnetic fields captured by the receiving coils 21, the processing unit is configured to calculate the position and/or orientation of the set of receiving coils 21 with respect to the set of transmitting coils 11, that is to say the position and/or orientation of the receiver 20 with respect to the transmitter 10.

The steps of the method for electromagnetically localizing a receiver 20 with respect to a transmitter 10 according to the first aspect and according to the second aspect will be disclosed in further details below.

Emitting a respective magnetic field

For each of the method according to the first aspect and according to the second aspect, the step of emitting E1 a respective magnetic field is performed by each magnetic field generator 11 of the transmitter 10.

When a voltage is imposed on the terminals of a magnetic field generator 11, or transmitting coil, of the transmitter 10, an electric current flows in the transmitting coil 11 which then generates a magnetic field proportional to the current flowing through it and the shape of which depends on the characteristics of the transmitting coil 11 (orientation, magnetic moment, shape ....).

Receiving and measuring each emitted magnetic field

For each of the method according to the first aspect and according to the second aspect, the step of receiving and measuring E2 each magnetic field emitted by the respective magnetic field generators 11 of the transmitter 10 is performed by the receiver 20.

In the presence of a variable magnetic field, a voltage proportional to the variation in the flux of the magnetic field appears in the receiving coil 21 of the receiver 20.

By measuring the voltage at the terminals of the receiving coil 21, using a voltmeter or other similar means, or by measuring the current passing through the receiving coil 21 by an ammeter or other similar means, it is possible to determine the magnetic field to which the receiving coil 21 is subjected, provided that the characteristics of the receiving coil 21 are known, which include notably the magnetic moments of the receiving coil 21, or at least the ratio of the magnetic moments of the receiving coil 21.

Method according to the first aspect

The method according to the first aspect is illustrated by way of a non-limiting example in figure 4 and may be performed by the processing unit.

In order to implement the method according to the first aspect, the transmitter 10 comprises at least four, for example six, non-coplanar magnetic field generators 11 separated from each other by known distances. The receiver 20 is preferably a tri-axis orthonormal receiver with approximately concentric receiving coils 21, thus the calculated respective norms do not depend on an orientation of the receiver 20.

The distances between each magnetic field generator 11 of the transmitter 10 and the receiver 20 may be identical or different, as illustrated by way of a non-limiting example in figure 3. The respective norms for each respective measured magnetic field are calculated in step E31 taking into account the known distances between the magnetic field generators 11, in order to accurately associate a respective norm with a corresponding distance between the corresponding magnetic field generator 11 and the receiver 20.

More specifically, estimating the preliminary position of the receiver 20 in step E32 may comprise running a multilateration algorithm on the calculated norms of the measured magnetic fields. A transmitter 10 comprising at least four magnetic field generators 11 allows the multilateration algorithm to have one unique solution. The multilateration algorithm allows for estimating the position of an object by measuring the difference in distances from multiple reference points and solving a system of equations, for example using numerical methods like least squares. The multilateration algorithm can be run accurately, as the distances separating the magnetic field generators 11 of the transmitter 10 are known.

More specifically, for the considered distances between the magnetic field generator 11 and the receiver 20, the norm of the respective measured magnetic field decreases proportionally to $1/r^3$, r corresponding to the distance between the corresponding magnetic field generator 11 and the receiver 20. Therefore, for a given opposing pair of magnetic field generators 11 separated from each other by a known distance, $(\frac{B1}{B2})^{\frac{1}{3}} = \frac{r2}{r1}$,

where B1 is the norm of the first measured magnetic field, B2 is the norm of the second measured magnetic field, r1 is the distance between the first magnetic field generator 11 and the receiver 20, and r2 is the distance between the second magnetic field generator 11 and the receiver 20. Consequently, $r2 = (\frac{B1}{B2})^{\frac{1}{3}} * r1$. Thus, for

example in the case where the transmitter 10 comprises six magnetic field generators 11, six such equations can be obtained and injected in a multilateration algorithm in order to estimate in step E32 the preliminary position of the receiver 20.

Calculating E5 the position of the receiver 20 based on each measured magnetic field and on the estimated preliminary position may be performed by the processing unit.

Calculating E5 the position of the receiver 20 may comprise running a minimization algorithm starting at the estimated preliminary position of the receiver 20. The minimization algorithm goes through iterations in order to converge towards the best possible solution, which corresponds to a minimum, which is only a local minimum in the case of the mirror position of the receiver 20, and is the global minimum in the case of the real position of the receiver 20. Here, as the estimated preliminary position of the receiver 20 is located in the hemisphere where the real position of the receiver 20 is located, the minimization algorithm will converge in step E5 with reliability towards the global minimum, that is to say the real position of the receiver 20.

The minimization algorithm can, for example, be a Levenberg-Marquardt or GaussNewton algorithm.

The method according to the first aspect may be for electromagnetically localizing a receiver 20 with respect to several transmitters 10. Each of the several transmitters 10 comprises at least four non-coplanar magnetic field generators 11 separated from each other by known distances. The steps of calculating E31 the respective norm and of calculating E32 the preliminary position are performed for each of the several transmitters 10. The position of the receiver 20 may be calculated based on each measured magnetic field and on one or several or each of the estimated preliminary positions. Each of the several transmitters 10 may emit E1 the respective magnetic fields by each of their magnetic field generators 11 simultaneously. The receiver 20 may receive and measure E2 each emitted magnetic field simultaneously. The receiver 20 may be able to discriminate which magnetic field is emitted by which of the several transmitters 10. For each given transmitter 10 among the several transmitters 10, the receiver 20 may independently calculate E31 the respective norm and may independently calculate E32 the preliminary position of the receiver 20 with respect to the given transmitter 10. The preliminary position of the receiver 20 associated with the given transmitter 10 is representative of which hemisphere of the given transmitter 10 the real position of the receiver 20 is located in and is also representative of a distance between the receiver 20 and the given transmitter 10. The respective positions of the transmitters 10 relative to each other do not need to be known prior to the implantation of the method according to the first aspect. The respective positions of the transmitters 10 relative to each other may be deduced from the position of the receiver 20 calculated in step E5.

Method according to the second aspect

The method according to the second aspect is illustrated by way of a non-limiting example in figure 5 and may be performed by the processing unit.

In order to implement the method according to the second aspect, the transmitter 10 comprises at least three, for example six, magnetic field generators 11. The receiver 20 can be a tri-axis orthonormal receiver, or another type of receiver.

The first initial position hypothesis H1 may be a position chosen randomly in a working area around the transmitter 10, that is to say in an area corresponding to a distance comprised between a minimal distance and a maximal distance around the transmitter 10.

Calculating E41 the first position P1 of the receiver 20 may comprise running a minimization algorithm starting at the first initial position hypothesis H1. The first position P1 is calculated based on each measured magnetic field. The minimization algorithm may be similar as the algorithm disclosed above used to calculate the position of the receiver 20 in step E5 of the method according to the first aspect. The minimization algorithm starts at the first position hypothesis H 1 and goes through iterations in order to converge towards the best possible solution corresponding to the first position P1 of the receiver 20, which corresponds to a minimum, which is only a local minimum in the case of the mirror position of the receiver 20, and is the global minimum in the case of the real position of the receiver 20. The minimization algorithm can, for example, be a Levenberg-Marquardt or GaussNewton algorithm.

The first residual may be calculated in step E43 as corresponding to a remaining error after optimization with the minimization algorithm, between the real measured magnetic fields received by the receiver 20, and the theoretical magnetic fields that would be measured by the receiver 20 in the first position P1 according to the model computation.

The second initial position hypothesis H2 is the mirror image of the first position P1 of the receiver 20 calculated in step E41. Therefore, one of the first position P1 of the receiver 20 and the second initial position hypothesis H2 is located in the hemisphere where the real position of the receiver 20 is located, while the other is located in the hemisphere where the mirror position of the receiver 20 is located.

Calculating E42 the second position P2 of the receiver 20 may be comprise running a minimization algorithm starting at the second initial position hypothesis H2. The first position P1 is calculated based on each measured magnetic field. The minimization algorithm may be similar as the algorithm disclosed above used to calculate the position of the receiver 20 in step E5 of the method according to the first aspect. The minimization algorithm starts at the second position hypothesis H2 and goes through iterations in order to converge towards the best possible solution corresponding to the second position P2 of the receiver 20, which corresponds to a minimum, which is only a local minimum in the case of the mirror position of the receiver 20, and is the global minimum in the case of the real position of the receiver 20. The minimization algorithm can, for example, be a Levenberg-Marquardt or GaussNewton algorithm.

The second residual may be calculated in step E44 as corresponding to a remaining error after optimization with the minimization algorithm, between the real measured magnetic fields received by the receiver 20, and the theoretical magnetic fields that would be measured by the receiver 20 in the second position P2 according to the model computation.

The residual is an indicator of the accuracy of the localization. The lower the residual, the more precise the localization. The lowest of the first residual and second residual is associated with the initial position hypothesis H1, H2 of the receiver 20 which is located in the hemisphere where the real position of the receiver 20 is located. The highest of the first residual and second residual is associated with the initial position hypothesis among the first initial position hypothesis H1 and the second initial position hypothesis H2 of the receiver 20 which is located in the hemisphere where the mirror position of the receiver 20 is located. The highest residual means that the minimization algorithm converged towards a local minimum, wherein the smallest residual means that the minimization algorithm converged towards the global minimum.

The initial position hypothesis H1, H2 associated with the smallest of the first residual and the second residual is thus selected in step E45. For example, if the first residual is smaller than the second residual, then the first initial position hypothesis H1 is selected in step E45 as corresponding to the preliminary position of the receiver 20.

This selection E45 of the first initial position hypothesis H1 or second initial position hypothesis H2 ensures that the preliminary position of the receiver 20 is positioned in the hemisphere where the real position of the receiver 20 is located.

Electromagnetically localizing several receivers

Each of the method according to the first aspect and the method according to the second aspect can be implemented for electromagnetically localizing several receivers 20 with respect to a transmitter 10. Each receiver 20 then receives and measures E2 each emitted magnetic field.

For the method according to the first aspect, a preliminary position is estimated for each respective receiver 20 and a position of each respective receiver 20 is calculated E5 based on each measured magnetic field and on each respective estimated preliminary position.

For the method according to the second aspect, the first position P1 of each respective receiver 20 is calculated E41 based on each measured magnetic field and on the first initial position hypothesis H1 and the second position P2 of each respective receiver 20 is calculated E42 based on each measured magnetic field and on the second initial position hypothesis H2.

Obviously, the present disclosure cannot be limited to the means and configuration described and illustrated herein, and it also extends to any equivalent means or configurations and to any technically operative combination of such means. In particular, the shape and arrangement of the transmitter and of the receiver, as well as the order and nature of the steps of the method for electromagnetically localizing the receiver, can be modified insofar as they fulfil the functionalities described in the present document.

**Claims**

1. A method for electromagnetically localizing a receiver (20) with respect to a transmitter (10), comprising:

   - emitting (E1) a respective magnetic field by each magnetic field generator (11) of the transmitter (10), said transmitter (10) comprising at least four non-coplanar magnetic field generators (11) separated from each other by known distances;
   - receiving and measuring (E2) each emitted magnetic field using the receiver (20);
   - calculating (E31) a respective norm for each respective measured magnetic field, each respective norm being representative of a distance between the receiver (20) and the respective magnetic field generator (11);
   - estimating (E32) a preliminary position of the receiver (20) based on the calculated respective norms; and
   - calculating (E5) a position of the receiver (20) based on each measured magnetic field and on the estimated preliminary position.

2. A method for electromagnetically localizing a receiver (20) with respect to a transmitter (10), comprising:

- emitting (E1) a respective magnetic field by each magnetic field generator (11) of the transmitter (10), said transmitter (10) comprising at least three non-coplanar magnetic field generators (11) separated from each other by known distances;
- receiving and measuring (E2) each emitted magnetic field using the receiver (20);
- calculating (E41) a first position (P1) of the receiver (20) based on each measured magnetic field and on a first initial position hypothesis (H1);
- calculating (E43) a first residual corresponding to the first position (P1);
- calculating (E42) a second position (P2) of the receiver (20) based on each measured magnetic field and on a second initial position hypothesis (H2), wherein the second initial position hypothesis (H2) corresponds to a mirror image of the first position (P1) of the receiver (20) calculated in step (E41);
- calculating (E44) a second residual corresponding to the second position (P2); and
- selecting (E45) the position (P1, P2) of the receiver (20) associated with the smallest of the first residual and the second residual, as corresponding to the calculated position of the receiver (20).

3. The method according to claim 1 or claim 2, wherein the receiver (20) is a tri-axis orthogonal receiver.

4. The method according to any one of claims 1 to 3, wherein the receiving coils (21) of the receiver (20) are approximately concentric.

5. The method according to any one of claims 1 to 4, wherein the transmitter (10) comprises at least a pair of two magnetic field generators (11) positioned symmetrically with respect to a center of the transmitter (10).

6. The method according to any one of claims 1 to 5, wherein the transmitter (10) comprises six magnetic field generators (11).

7. The method according to claim 6, wherein the transmitter (10) further comprises a housing (12) in the form of a rectangular block and wherein the six magnetic field generators (11) of the transmitter (10) are arranged in three orthogonal opposing pairs, each magnetic field generator (11) being positioned facing a respective side of the rectangular block of the housing (12).

8. The method according to claim 7, wherein the housing (12) of the transmitter (10) is in the form of a cube.

9. The method according to any one of claims 1 to 8, wherein each known distance separating two magnetic field generators (11) is at least equal to 0,5 cm, for example may be comprised between 1 cm and 5 cm, for example may be equal to 3 cm.

10. The method according to claim 1, wherein estimating the preliminary position of the receiver (20) comprises running a multilateration algorithm on the calculated norms of the measured magnetic fields.

11. The method according to claim 1, wherein calculating (E5) the position of the receiver (20) comprises running a minimization algorithm starting at the estimated preliminary position of the receiver (20).

12. The method according to claim 1, for electromagnetically localizing several receivers (20) with respect to a transmitter (10), wherein each receiver (20) receives and measures (E2) each emitted magnetic field, wherein a preliminary position is estimated for of each respective receiver (20) and wherein a position of each respective receiver (20) is calculated (E5) based on each measured magnetic field and on each respective estimated preliminary position.

13. The method according to claim 1, for electromagnetically localizing a receiver (20) with respect to several transmitters (10), wherein each of the several transmitters (10) comprises at least four non-coplanar magnetic field generators (11) separated from each other by known distances, and wherein the steps of calculating (E31) the respective norm and of calculating (E32) the preliminary position are performed for each of the several transmitters (10).

14. The method according to claim 2, wherein calculating (E41) the first position (P1) of the receiver (20) comprises running a minimization algorithm starting at the first initial position hypothesis (H1) and calculating (E42) the second position (P2) of the receiver (20) comprises running a minimization algorithm starting at the second initial position hypothesis (H2).

15. The method according to claim 2, for electromagnetically localizing several receivers (20) with respect to a transmitter (10), wherein each receiver (20) receives and measures (E2) each emitted magnetic field, wherein the first position (P1) of each respective receiver (20) is calculated (E41) based on each measured magnetic field and on the first initial position hypothesis (H1) and wherein the second position (P2) of each respective receiver (20) is calculated (E42) based on each measured magnetic field and on the second initial position hypothesis (H2).

FIG. 2

FIG. 1

FIG. 3

EP 4 764 588 A1

## FIG. 4

| | |
|---|---|
| Emitting respective magnetic fields | E1 |
| Receiving and measuring the magnetic fields | E2 |
| Calculating norms for the measured magnetic fields | E31 |
| Estimating the preliminary position of the receiver | E32 |
| Calculating a position of the receiver | E5 |

## FIG. 5

| | |
|---|---|
| Emitting respective magnetic fields | E1 |
| Receiving and measuring the magnetic fields | E2 |
| 1st position hypothesis | H1 |
| Calculating a 1st position P1 of the receiver | E41 |
| Calculating a 1st residual associated with the first position P1 | E43 |
| Calculating a 2nd position hypothesis (mirror of the 1st position hypothesis) | H2 |
| Calculating a 2nd position P2 of the receiver | E42 |
| Calculating a 2nd residual associated with the second position P2 | E44 |
| Selecting the calculated position of the receiver as the one associated with the smallest residual | E45 |

Europäisches
Patentamt

European
Patent Office

Office européen
des brevets

## EUROPEAN SEARCH REPORT

**Application Number**

EP 24 30 7139

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 887 309 B1 (BIOSENSE WEBSTER INC [US]) 6 July 2016 (2016-07-06) * paragraphs [0007], [0042], [0047] * ----- | 1,3-13 | INV. G01S5/16 A61B5/06 |
| A | FREDERICK H RAAB ET AL: "Magnetic Position and Orientation Tracking System", IEEE TRANSACTIONS ON AEROSPACE AND ELECTRONIC SYSTEMS, IEEE SERVICE CENTER, PISCATAWAY, NJ, US, vol. AES-15, no. 5, 1 September 1979 (1979-09-01), pages 709-717, XP001348413, ISSN: 0018-9251 * Section II - Magnetic-Field Coupling; page 709 - page 717 * ----- | 1-15 | ADD. A61B34/20 G01V3/10 |
| A | US 2014/051983 A1 (SCHROEDER TOBIAS [US] ET AL) 20 February 2014 (2014-02-20) Describes a magnetic localization system focused on ambiguity resolution using 3-axis and extra receiver coils. Emphasizes vector component measurement. Norm-based estimation not disclosed.; * abstract * ----- | 1-15 | |
| A | US 8 180 430 B2 (GOVARI ASSAF [IL]; BAR-TAL MEIR [IL] ET AL.) 15 May 2012 (2012-05-15) * abstract * ----- | 1-15 | TECHNICAL FIELDS SEARCHED (IPC) G01S G01V A61B |

The present search report has been drawn up for all claims

| Place of search | Date of completion of the search | Examiner |
|---|---|---|
| Munich | 17 July 2025 | Chindamo, Gregorio |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 764 588 A1

Europäisches
Patentamt

European
Patent Office

Office européen
des brevets

**Application Number**

EP 24 30 7139

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

   see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION**
**SHEET B**

Application Number

EP 24 30 7139

The Search Division considers that the present European patent application does not comply with the requirements of unity of invention and relates to several inventions or groups of inventions, namely:

1. claims: 1, 3-13

   How to estimate the receiver's position in a manner that supports improved robustness of final position calculation.
   ---

2. claims: 2-9, 14, 15

   How to resolve ambiguity between symmetric position estimates resulting from magnetic field-based localization.
   ---

## ANNEX TO THE EUROPEAN SEARCH REPORT
## ON EUROPEAN PATENT APPLICATION NO.

EP 24 30 7139

This annex lists the patent family members relating to the patent documents cited in the above-mentioned European search report.
The members are as contained in the European Patent Office EDP file on
The European Patent Office is in no way liable for these particulars which are merely given for the purpose of information.

17-07-2025

| Patent document cited in search report | | Publication date | Patent family member(s) | | Publication date |
|---|---|---|---|---|---|
| EP 1887309 | B1 | 06-07-2016 | AU | 2007203658 A1 | 21-02-2008 |
| | | | BR | PI0703316 A | 08-04-2008 |
| | | | CA | 2596404 A1 | 07-02-2008 |
| | | | CN | 101120877 A | 13-02-2008 |
| | | | CN | 102210611 A | 12-10-2011 |
| | | | EP | 1887309 A1 | 13-02-2008 |
| | | | EP | 2256454 A1 | 01-12-2010 |
| | | | EP | 2259008 A1 | 08-12-2010 |
| | | | IL | 185005 A | 30-05-2013 |
| | | | JP | 5191704 B2 | 08-05-2013 |
| | | | JP | 2008062040 A | 21-03-2008 |
| | | | KR | 20080013725 A | 13-02-2008 |
| | | | US | 2008033282 A1 | 07-02-2008 |
| | | | US | 2011251814 A1 | 13-10-2011 |
| | | | US | 2011251815 A1 | 13-10-2011 |
| US 2014051983 | A1 | 20-02-2014 | NONE | | |
| US 8180430 | B2 | 15-05-2012 | AT | E554700 T1 | 15-05-2012 |
| | | | AU | 2007200938 A1 | 20-09-2007 |
| | | | BR | PI0700635 A | 06-11-2007 |
| | | | CA | 2579542 A1 | 03-09-2007 |
| | | | CA | 2858896 A1 | 03-09-2007 |
| | | | CN | 101028188 A | 05-09-2007 |
| | | | CN | 101803924 A | 18-08-2010 |
| | | | EP | 1829477 A2 | 05-09-2007 |
| | | | IL | 181676 A | 27-02-2014 |
| | | | JP | 5265122 B2 | 14-08-2013 |
| | | | JP | 2007236937 A | 20-09-2007 |
| | | | KR | 20070090813 A | 06-09-2007 |
| | | | US | 2006241394 A1 | 26-10-2006 |
| | | | US | 2006241397 A1 | 26-10-2006 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8180430 B2

**Non-patent literature cited in the description**

- **FREDERICK H. RAAB et al.** Magnetic Position and Orientation Tracking System. *IEEE Transactions on Aerospace and Electronic Systems*, September 1979, vol. AES-15 (5)

- La localisation spatiale d'outils chirurgicaux par systemes electromagnetiques alternatifs. Applications et domaines de validite des modelisations numeriques. **JOFFREY PAILLE**. Thesis. Universit6 Joseph-Fourier - Grenoble, 2004, vol. I